(19) Europäisches Patentamt
European Patent Office
Office européen des brevets

(11) Numéro de publication : **0 332 484 B1**

# (12) FASCICULE DE BREVET EUROPEEN

(45) Date de publication du fascicule du brevet : **06.05.92 Bulletin 92/19**

(51) Int. Cl.[5] : **C07C 407/00**

(21) Numéro de dépôt : **89400416.7**

(22) Date de dépôt : **15.02.89**

## (54) Procédé de préparation de peroxydes terpéniques, les nouveaux peroxydes ainsi obtenus et leur emploi.

(30) Priorité : **18.02.88 FR 8801920**

(43) Date de publication de la demande : **13.09.89 Bulletin 89/37**

(45) Mention de la délivrance du brevet : **06.05.92 Bulletin 92/19**

(84) Etats contractants désignés : **AT BE CH DE ES FR GB GR IT LI LU NL SE**

(56) Documents cités :
**GB-A- 767 615**
**US-A- 2 516 649**
**J. ORG. CHEM., vol. 51, 1986, pages 1790-1793, American Chemical Society; J.J. BLOODWORTH et al.: "Alkylated perepoxides:Peroxonium vs. phenonium intermediates from beta-haloalkyl tert-butyl peroxides and silver trifluoroacetate"**

(56) Documents cités :
**TETRAHEDRON, vol. 41, no. 19, 1985, pages 4047-4056, Pergamon Press Ltd, GB; B. MAILLARD et al.: "Intramolecular homolyticdisplacements 7 - free radical additions to unsaturated peroxy compounds: synthesis of oxygenated heterocycles"**
**J. OF ORGANIC CHEMISTRY OF THE USSR, vol. 23, no. 9, partie 1, septembre 1987, pages 1640-1642, Plenum Publishing Corp.;YU.V. PANCHENKO et al.: "Synthesis and reactions of peroxides of the enyne series"**

(73) Titulaire : **RHONE-POULENC SANTE
20, avenue Raymond Aron
F-92160 Antony (FR)**

(72) Inventeur : **Mercier, Claude
85 avenue du Point du Jour
F-69005 Lyon (FR)**

(74) Mandataire : **Le Pennec, Magali et al
RHONE-POULENC SANTE, Service Brevets,
20 Avenue Raymond Aron
F-92165 Antony Cédex (FR)**

**EP 0 332 484 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de peroxydes terpéniques de formule générale :

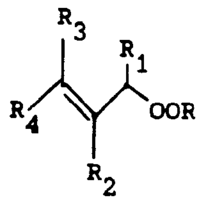

(I)

dans laquelle R représente un radical alkyle tertiaire éventuellement substitué par un ou plusieurs radicaux phényles tel qu'un radical tertiobutyle, trityle, un radical cuményle, un radical cycloalkyle tel que cyclohexyle ou un radical trialkylsilyle, tel que le radical triméthylsilyle, $R_1$, $R_2$ et $R_3$, identiques ou différents, représentent un atome d'hydrogène ou un radical aliphatique contenant 1 à 20 atomes de carbone et éventuellement une ou plusieurs doubles ou triples liaisons et pouvant être substitué par un ou plusieurs radicaux fonctionnels inertes, identiques ou différents, tels que, par exemple, les radicaux nitro ou dialkylamino, et $R_4$ représente un radical aliphatique contenant 1 à 20 atomes de carbone et éventuellement une plus plusieurs doubles ou triples liaisons et pouvant être substitué par un ou plusieurs radicaux fonctionnels inertes identiques ou différents tels que, par exemple, les radicaux nitro ou dialkylamino, ou bien
$R_3$ représente un atome d'hydrogène et $R_2$ et $R_4$ forment ensemble un radical alkylène contenant 3 ou 4 atomes de carbone éventuellement substitué par un ou plusieurs radicaux méthyles.

Il est connu de préparer des peroxydes organiques mixtes par action d'un peroxyde de métal alcalin sur un dérivé halogéné en opérant dans un alcool tel que le méthanol, l'éthanol ou l'isopropanol [brevet américain US 2 403 709 ; Campbell et al, J. Amer. Chem. Soc, 1788 (1955) ; R. Hiatt et coll., Can. J. Chem., 450 (1980)] ou dans un système biphasique par transfert de phase [B. Maillard et coll., Tetrahedron, 5309 (1986) ; A.A. Turovskü et coll., Zhurh. Obschei. Khim., 43, 1167 (1973)]. Cependant la mise en oeuvre de ces procédés conduit aux peroxydes mixtes avec des rendements médiocres et s'accompagne fréquemment de la formation de produits secondaires indésirables.

Il a maintenant été trouvé, et c'est ce qui fait l'objet de la présente invention, que les peroxydes de formule générale (I) peuvent être obtenus par action d'un hydroperoxyde de formule générale :

R-OOH (II)

dans laquelle R est défini comme précédemment, sur un produit de formule générale :

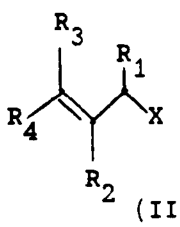

(IIIa) ou

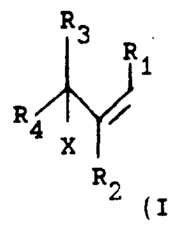

(IIIb)

dans laquelle les symboles $R_1$, $R_2$, $R_3$ et $R_4$ sont définis comme précédemment et X représente un atome ou radical remplaçable par substitution nucléophile tel que, par exemple, un atome d'halogène (chlore ou brome) ou un radical alcanoyloxy (acétyloxy) ou un radical alkyl ou arylsulfonyloxy (méthanesulfonyloxy ou p.toluènesulfonyloxy) ou un radical $-ONO_2$ ou

$$-O\underset{\underset{O}{\|}}{P}(OR')_2$$

en opérant dans un solvant aprotique polaire en présence d'une base minérale.

Généralement, le procédé est mis en oeuvre en ajoutant le produit de formule générale (IIIa) ou (IIIb) à une solution d'un hydroperoxyde de formule générale (II) dans un mélange homogène d'un solvant aprotique polaire et d'eau en présence d'une base minérale et en opérant à une température comprise entre 0 et 50°C, de préférence, voisine de 20°C.

Plus particulièrement, les peroxydes de formule générale (I) sont obtenus en ajoutant le produit de formule générale (IIIa) ou (IIIb) à une solution d'un hydroperoxyde de formule générale (II) dans un mélange homogène d'un solvant organique aprotique polaire, tel que le sulfolane, et d'eau en présence d'une base minérale telle que la potasse à une température comprise entre 0 et 50°C, et de préférence, voisine de 20°C.

La présente invention concerne également les produits de formule générale (I) dans laquelle R, $R_1$, $R_2$, $R_3$ et $R_4$ sont définis comme précédemment, à l'exclusion des produits de formule générale (I) dans laquelle R représentant un radical tertiobutyle et $R_1$ représentant un atome d'hydrogène,

– $R_2$ représente un atome d'hydrogène et $R_3$ et $R_4$ représentent chacun un radical méthyle, ou bien

– $R_2$ représente un radical méthyle, $R_3$ représente un atome d'hydrogène et $R_4$ représente un radical méthyle ou éthyle.

Les peroxydes de formule générale (I) sont des intermédiaires particulièrement utiles en synthèse terpénique et ils permettent, par exemple, d'accéder à des intermédiaires pour la préparation des vitamines A et E ou pour la préparation de parfums.

Ainsi les produits de formule générale (I) dans laquelle $R_1$ et $R_2$ représentent un atome d'hydrogène, $R_3$ représente un radical méthyle et $R_4$ représente un radical méthyl-4 pentène-3 yle, c'est-à-dire les peraxydes de géranyle, peuvent être utilisés pour préparer le citral après chauffage en présence d'une base organique ou le tétrahydrogéraniol après hydrogénation totale.

Les exemples suivants, donnés à titre non limitatif, montrent comment l'invention peut être mise en pratique.

EXEMPLE 1

Dans un ballon tricol de 250 cm3, on introduitt 40 cm3 de sulfolane et 6,5 g de potasse en pastilles. Après refroidissement à 10°C, on ajoute goutte à goutte une solution aqueuse d'hydroperoxyde de tertiobutyle à 70 % (12,9 g). On maintient pendant 2 heures à 20°C puis on ajoute goutte à goutte, à une température comprise entre 10 et 20°C, 17,2 g de chlorure de géranyle (0,1 mole). On maintient pendant 16 heures à 20°C. Après extraction par le pentane et purification du produit obtenu par flash chromatographie, on obtient 12 g de peroxyde de géranyle et de tertiobutyle dont la structure est confirmée par le spectre infra-rouge, le spectre de masse et le spectre de résonance magnétique nucléaire du proton.

Le taux de transformation du chlorure de géranyle est voisin de 100 % et le rendement en peroxyde de géranyle et de tertiobutyle est voisin de 53 %.

EXEMPLE 2

Dans un ballon de 25 cm3, on introduit 1,13 g de peroxyde de géranyle et de tertiobutyle (5 mmoles) et 5 cm3 de n.trioctylamine. On chauffe le mélange pendant 9 heures à 100°C. Après refroidissement, l'analyse du mélange réactionnel par chromatographie en phase vapeur montre que :

– le taux de transformation du peroxyde de géranyle et de tertiobutyle est voisin de 92 %,

– le rendement en citral est voisin de 12,6 % par rapport au peroxyde de géranyle et de tertiobutyle transformé.

EXEMPLE 3

Dans un autoclave, on introduit 2,26 g de peroxyde de géranyle et de tertiobutyle (10 mmoles) en solution dans 40 cm3 d'éthanol à 95 %, et 100 mg de palladium sur charbon à 10 % de palladium. On agite pendant 4 heures sous une pression de 20 bars d'hydrogène. Après dégazage et filtration, l'analyse du mélange réactionnel par CPV montre que :

– le taux de transformation du peroxyde de géranyle et de tertiobutyle est voisin de 100 %,

– le rendement en tétrahydrogéraniol est voisin de 72 %.

EXEMPLES 4 A 7

On opère comme dans l'exemple 1 mais à partir de bromo-1 méthyl-3 butène-2 et de différents hydroperoxydes. Les résultats sont rassemblés dans le tableau 1.

**TABLEAU 1**

| Exemple | Hydroperoxyde | Rendement (%) en peroxyde mixte isolé |
|---|---|---|
| 4 | $(CH_3)_3C-O-OH$ | 41 % |
| 5 | $C_6H_5-C(CH_3)_2-O-OH$ | 54 % |
| 6 | 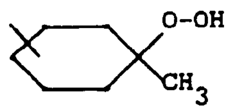 | 37 % |
| 7 | $(C_6H_5)_3C-O-OH$ | 46 % |

EXEMPLE 8

En opérant comme dans l'exemple 1 en remplaçant le chlorure de géranyle par le bromure de géranyle, on obtient le peroxyde de géranyle et de tertiobutyle avec un rendement de 50 %.

## Revendications

1. Procédé de préparation de peroxydes terpéniques de formule générale :

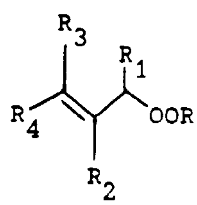

dans laquelle :

R représente un radical alkyle tertiaire éventuellement substitué par un ou plusieurs radicaux phényles, un radical cycloalkyle ou un radical trialkylsilyle, $R_1$, $R_2$ et $R_3$, identiques ou différents, représentent un atome d'hydrogène ou un radical aliphatique contenant 1 à 20 atomes de carbone et éventuellement une ou plusieurs doubles ou triples liaisons et pouvant être substitué par un ou plusieurs radicaux fonctionnels inertes identiques ou différents et $R_4$ représente un radical aliphatique contenant 1 à 20 atomes de carbone et éventuellement une ou plusieurs doubles ou triples liaisons et pouvant être substitué par un ou plusieurs radicaux fonctionnels inertes identiques ou différents, ou bien

$R_3$ représente un atome d'hydrogène et $R_2$ et $R_4$ forment ensemble un radical alkylène contenant 3 ou 4 atomes de carbone et éventuellement substitué par un ou plusieurs radicaux méthyles, caractérisé en ce que l'on fait réagir un hydroperoxyde de formule générale :

ROOH

dans laquelle R est défini comme dans la revendication 1 avec un produit de formule générale :

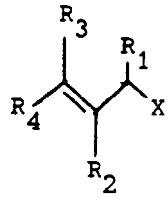

ou

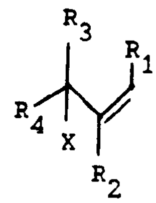

dans laquelle $R_1$, $R_2$, $R_3$ et $R_4$ sont définis comme dans la revendication 1 et X représente un atome ou radical remplaçable par substitution nucléophile en opérant dans un solvant aprotique polaire en présence d'une base minérale à une température comprise entre 0 et 50°C.

2. Procédé selon la revendication 1 caractérisé en ce que l'atome ou le radical remplaçable par substitution nucléophile est choisi parmi les atomes d'halogène et les radicaux alcanoyloxy, alkylsulfonyloxy, arylsulfonyloxy, $-ONO_2$ ou

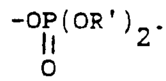

3. Procédé selon la revendication 1 caractérisé en ce que le solvant aprotique polaire est le sulfolane.

4. Procédé selon la revendication 1 caractérisé en ce que la base minérale est la potasse.

5. Les nouveaux peroxydes terpéniques de formule générale :

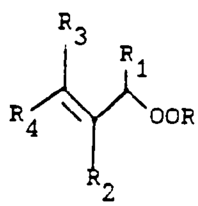

dans laquelle R, $R_1$, $R_2$, $R_3$ et $R_4$ sont définis comme dans la revendication 1, étant entendu que, lorsque R représente un radical tertiobutyle et $R_1$ représente un atome d'hydrogène,

- $R_2$ représentant un atome d'hydrogène, $R_3$ et $R_4$ ne peuvent pas représenter chacun un radical méthyle, ou bien
- $R_2$ représentant un radical méthyle, $R_3$ et $R_4$ ne peuvent pas représenter respectivement un atome d'hydrogène ou un radical méthyle ou éthyle.

## Patentansprüche

1. Verfahren zur Herstellung von Terpenperoxiden der allgemeinen Formel:

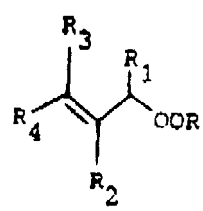

worin:

R einen gegebenenfalls durch einen oder mehrere Phenylreste substituierten tertiären Alkylrest, einen Cycloalkylrest oder einen Trialkylsilylrest bedeutet, $R_1$, $R_2$ und $R_3$, die gleich oder verschieden sind, ein Wasserstoffatom oder einen aliphatischen Rest mit 1 bis 20 Kohlenstoffatomen und gegebenenfalls einer oder mehreren Doppel- oder Dreifachbindungen bedeuten, der durch einen oder mehrere gleiche oder verschiedene, inerte, funktionelle Reste substituiert sein kann, und $R_4$ einen aliphatischen Rest mit 1 bis 20 Kohlenstoffatomen

und gegebenenfalls einer oder mehreren Doppel- oder Dreifachbindungen bedeutet, der durch einen oder mehrere gleiche oder verschiedene, inerte, funktionelle Reste substituiert sein kann, oder

$R_3$ ein Wasserstoffatom bedeutet und $R_2$ und $R_4$ zusammen einen Alkylenrest mit 3 oder 4 Kohlenstoffatomen bilden, der gegebenenfalls durch einen oder mehrere Methylreste substituiert sein kann, dadurch gekennzeichnet daß man ein Hydroperoxid der allgemeinen Formel:

ROOH

in welcher R wie in Anspruch 1 definiert ist, mit einem Produkt der allgemeinen Formel:

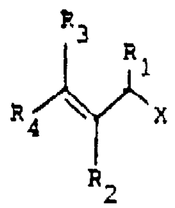

oder

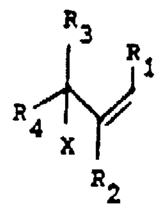

in welcher $R_1$, $R_2$, $R_3$ und $R_4$ wie in Anspruch 1 definiert sind und X ein Atom oder einen Rest bedeutet, die durch nukleophile Substitution ersetzbar sind, wobei man in einem aprotischen, polaren Lösungsmittel in Gegenwart einer anorganischen Base bei einer Temperatur zwischen 0 und 50°C arbeitet.

2. Verfahren nach Anspruch 1, dadurch gekennzeichnet, daß das Atom oder der Rest, die durch nukleophile Substitution ersetzbar sind, ausgewählt ist aus den Halogenatomen und den Alkanoyloxy-, Alkylsulfonyloxy-, Arylsulfonyloxy-, $-ONO_2$ oder

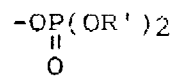

Resten.

3. Verfahren nach Anspruch 1, dadurch gekennzeichnet, daß das aprotische, polare Lösungsmittel Sulfolan ist.

4. Verfahren nach Anspruch 1, dadurch gekennzeichnet, daß die anorganische Base Kaliumhydroxid ist.

5. Neue Terpenperoxide der allgemeinen Formel:

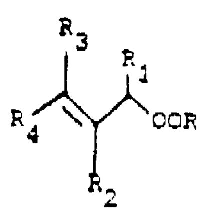

in welcher R, $R_1$, $R_2$, $R_3$ und $R_4$ wie in Anspruch 1 definiert sind, wobei selbstverständlich, wenn R einen Tertiobutylrest bedeutet und $R_1$ ein Wasserstoffatom bedeutet,

– $R_2$ ein Wasserstoffatom bedeutet, $R_3$ und $R_4$ nicht jeweils einen Methylrest bedeuten können, oder

– $R_2$ einen Methylrest bedeutet, $R_3$ und $R_4$ nicht ein Wasserstoffatom bzw. einen Methyl- oder Äthylrest bedeutet können.

## Claims

1. Process for the preparation of terpene peroxides of general formula:

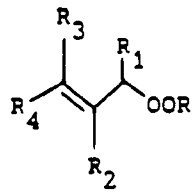

in which:

R represents a tertiary alkyl radical optionally substituted by one or more phenyl radicals, a cycloalkyl radical or a trialkylsilyl radical, $R_1$, $R_2$ and $R_3$, which may be identical or different, represent a hydrogen atom or an aliphatic radical containing 1 to 20 carbon atoms and optionally one or more double or triple bonds, which radical may be substituted by one or more identical or different inert functional radicals, and $R_4$ represents an aliphatic radical containing 1 to 20 carbon atoms and optionally one or more double or triple bonds, which radical may be substituted by one or more identical or different inert functional radicals, or

$R_3$ represents a hydrogen atom and $R_2$ and $R_4$ together form an alkylene radical containing 3 or 4 carbon atoms and optionally substituted by one or more methyl radicals, characterised in that a hydroperoxide of general formula:

ROOH

in which R is defined as in Claim 1, is reacted with a product of general formula:

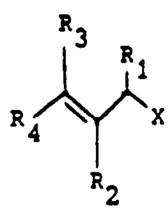 or 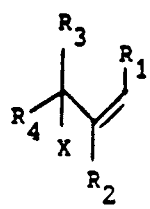

in which $R_1$, $R_2$, $R_3$ and $R_4$ are defined as in Claim 1 and X represents an atom or radical replaceable by nucleophilic substitution, by carrying out the reaction in a polar aprotic solvent in the presence of an inorganic base at a temperature of between 0 and 50°C.

2. Process according to Claim 1, characterised in that the atom or the radical replaceable by nucleophilic substitution is chosen from halogen atoms and alkanoyloxy, alkylsulphonyloxy, arylsulphonyloxy, $-ONO_2$ or

$$-O\underset{\underset{O}{\|}}{P}(OR')_2$$

radicals.

3. Process according to Claim 1, characterised in that the polar aprotic solvent is sulpholane.

4. Process according to Claim 1, characterised in that the inorganic base is potassium hydroxide.

5. The new terpene peroxides of general formula:

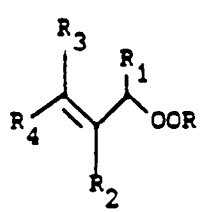

in which R, $R_1$, $R_2$, $R_3$ and $R_4$ are defined as in Claim 1, it being understood that if R represents a tert-butyl radical and $R_1$ represents a hydrogen atom,

– $R_2$ representing a hydrogen atom, $R_3$ and $R_4$ may not each represent a methyl radical, or

– $R_2$ representing a methyl radical, $R_3$ and $R_4$ may not represent, respectively, a hydrogen atom or a methyl or ethyl radical.